# EUROPEAN PATENT SPECIFICATION

(11) **EP 1 776 471 B1**
(45) Date of publication and mention of the grant of the patent: **03.07.2013**
(21) Application number: 05755025.3
(22) Date of filing: 25.05.2005
(51) Int. Cl.: C12Q 1/68, G01N 33/574, G01N 33/50

(54) **METHOD OF MEASURING CANCER SUSCEPTIBILITY**
VERFAHREN ZUR MESSUNG DER ANFÄLLIGKEIT FÜR KREBS
PROCEDE DE MESURE DE LA SUSCEPTIBILITE AU CANCER

(30) Priority: 25.05.2004 US 574248 P
(43) Date of publication of application: 25.04.2007
(73) Proprietor: Hitachi Chemical Company, Ltd., Tokyo 100-6606 (JP); HITACHI CHEMICAL RESEARCH CENTER, INC., Irvine, CA 92617 (US)
(72) Inventor: MITSUHASHI, Masato, Irvine, California 92614 (US)
(74) Representative: Thurston, Joanna
(86) International application number: PCT/US2005/018289
(87) International publication number: WO 2005/115115

(56) References cited:
- WO-A-03/030719
- WO-A-2006/045053
- US-A1- 2002 048 566
- US-A1- 2004 072 268
- AMUNDSON S A ET AL: "FLUORESCENT CDNA MICROARRAY HYBRIDIZATION REVEALS COMPLEXITY AND HETEROGENEITY OF CELLULAR GENOTOXIC STRESS RESPONSES" ONCOGENE, NATURE PUBLISHING GROUP, GB BASINGSTOKE, HANTS, vol. 18, no. 24, 17 June 1999 (1999-06-17), pages 3666-3672, XP000971604 ISSN: 0950-9232
- NAKANO K VOUSDEN K H: "Puma, a novel proapoptotic gene, is induced by p53" MOLECULAR CELL, CELL PRESS, CAMBRIDGE, MA, US, vol. 7, 1 March 2001 (2001-03-01), pages 683-694, XP002958900 ISSN: 1097-2765
- CHEN W ET AL: "Inhibition of ultraviolet B--induced c-fos gene expression and p38 mitogen-activated protein kinase activation by (-)-epigallocatechin gallate in a human keratinocyte cell line." MOLECULAR CARCINOGENESIS FEB 1999, vol. 24, no. 2, February 1999 (1999-02), pages 79-84, XP002548802 ISSN: 0899-1987
- ANDREW R SNYDER ET AL: "Gene expression profiling after irradiation: Clues to understanding acute and persistent responses?" CANCER AND METASTASIS REVIEWS, KLUWER ACADEMIC PUBLISHERS, DO, vol. 23, no. 3-4, 1 August 2004 (2004-08-01), pages 259-268, XP019205152 ISSN: 1573-7233
- HOQUE ET AL. CANCER LETTERS vol. 75, 2003, pages 75 - 80

## Description

### Field of the Invention

The present invention relates to a method of measuring susceptibility to cancer in individuals as defined in the claims. The invention further relates to a method of screening compounds for cancer prophylaxis effects in individuals as defined in the claims.

### Description of the Related Art

Cancer can develop as a result of DNA mutation in somatic cells due to a personal exposure to DNA-damaging agents. Such agents include radiation, chemicals, foods, and free radicals. Although most DNA damage is corrected by appropriate cellular responses, accumulation of multiple DNA damages at critical genomic sites can lead to cancer. Thus, cancer susceptibility is dependent on the balance between DNA damage and the corresponding cellular responses in each individual. Impaired DNA damage responses are known to induce cancer in high frequency in rare diseases, such as ataxia telangiectasia.

Amundson et. al., "Fluorescence cDNA Microarray Hybridization Reveals Complexity and Heterogeneity of Cellular Genotoxic Stress Response", Oncogene, Nature Publishing Group, Volume 18, 24, (1999), pages 3666-3672, describes the dependence of cell response to ionising radiation on changes in gene expression, in particular the response of genes in their p53 wild-type ML-1 human mycloid cell line were studied.

US 2002/048566, El-Deiry et. al. describes methods of modulating cellular apoptosis by increasing the difference in toxicity response between a target cell and a non-target cell. In particular, the difference between cancer and non-cancer cells is studied.

Nikano et. al., "PUMA, A Novel Proapoptotic Gene, Is Induced by p53", Molecular Cell, Volume 7, (2001), pages 683-694, describes the function of the p53 tumour-suppressor protein as a transcriptional activator. p53 inducible genes that play a role in the induction of apoptosis are also described, including PUMA as a target for activation by p53.

WO 03/030719, University of Chicago, describes a method and kit for use in selecting approaches to treating cancer, in particular, methods involving analysing the level of expression of one or more cancer-associated genes in a sample containing cancer cells and then selecting a type, schedule, route and/or amount of radiation therapy for treatment of the subject based upon these results are described.

US 2004/072268 discloses a method for identifying an anti-cancer agent wherein the ability of an agent to change cell survival rates in the presence of ionizing radiation is determined. The method may encompass the determination of the expression of p53 response elements.

### SUMMARY OF THE INVENTION

An embodiment of a method of determining susceptibility to cancer in an individual is defined in claim 1. Cells of the individual are exposed to a mutagenic stimulant in vitro, the level of the growth-suppressing marker in the exposed cells and in non-exposed cells of the individual is measured, and the individual's susceptibility to cancer is determined based on the difference in marker levels in the exposed and non-exposed cells.

The cells are derived from whole blood of the individual. In a further aspect of this embodiment, the levels measured are mRNA levels. In a further aspect of this embodiment, the individual is a human. The growth-suppressing marker is the cytostatic marker p21, the cytocidal marker, BAX, and/or the cytocidal marker PUMA.

In a further aspect of this embodiment, the mutagenic stimulant is ionizing radiation.

Disclosed is a method in which the susceptibility to cancer is determined to decrease when the level of the growth-suppressing marker strongly increases after exposure. In a further aspect of this embodiment, the susceptibility to cancer is determined to increase when the level of the growth-suppressing marker does not increase or weakly increases after exposure.

In a further aspect of this embodiment, the level of a plurality of growth-suppressing markers is measured. In a further aspect of this embodiment, the plurality of growth-suppressing markers includes at least one cytostatic marker and one cytocidal marker. In a further aspect of this embodiment, the cytostatic marker is p21 and the cytocidal marker is PUMA.

Another embodiment of the method of determining susceptibility to cancer in an individual is described in claim 8. A baseline average measurement of levels of a growth-suppressing marker in cells is obtained from a plurality of members of the individual's species after said cells had been exposed to a mutagenic stimulant in vitro, cells of the individual are exposed to a mutagenic stimulant in vitro, the level of the growth-suppressing marker in the cells is measured after exposure; and the individual's susceptibility to cancer is determined, wherein a higher level than the baseline average measurement indicates a lower risk of cancer and a lower level than the baseline average measurement indicates a higher risk of cancer.

The cells are derived from whole blood of the individual. In a further aspect of this embodiment, the levels measured are mRNA levels. In a further aspect of this embodiment, the individual is a human. The growth-suppressing marker is the cytostatic marker p21, the cytocidal marker BAX, and/or the cytocidal marker PUMA.

In a further aspect of this embodiment, the mutagenic stimulant is ionizing radiation. In a further aspect of this embodiment, the level of a plurality of growth-suppressing markers is measured. In a further aspect of this embodiment, the plurality of growth-suppressing markers includes at least one cytostatic marker and one cytocidal marker. In a further aspect of this embodiment, the cytostatic marker is p21 and the cytocidal marker is PUMA.

A method of screening a compound for cancer prophylaxis effects in an individual is described in claim 14. Cells of the individual are incubated with the compound, the incubated cells and non-incubated cells of the individual are exposed to a mutagenic stimulant in vitro, levels of a growth-suppressing marker are measured in the exposed cells and in non-incubated, non-exposed cells of the individual, and compounds having cancer prophylaxis effects are identified based on the difference in levels of the growth-suppressing marker in the incubated cells and the non-incubated cells after exposure. In an aspect of this embodiment, the levels of the growth-suppressing marker are measured in incubated cells that are not exposed to a mutagenic stimulant.

The cells are derived from whole blood of the individual. In a further aspect of this embodiment, the levels measured are mRNA levels. In a further aspect of this embodiment, the individual is a human. The growth-suppressing marker is the cytostatic marker p21, the cytocidal marker BAX, and/or the cytocidal marker PUMA. In a further aspect of this embodiment, the mutagenic stimulant is ionizing radiation. In a further aspect of this embodiment, the level of a plurality of growth-suppressing markers is measured. In a further aspect of this embodiment, the plurality of growth-suppressing markers includes at least one cytostatic marker and one cytocidal marker. In a further aspect of this embodiment, the cytostatic marker is p21 and the cytocidal marker is PUMA.

In a further aspect of this embodiment, compounds exhibiting a greater increase in post-exposure levels of the growth-suppressing marker in incubated cells than in non-incubated cells are identified as having cancer prophylaxis effects. In a further aspect of this embodiment, compounds exhibiting no or a small increase in levels of the growth-suppressing marker in incubated unexposed cells relative to unincubated, unexposed cells, and exhibiting a greater increase in post-exposure levels of the growth-suppressing marker in incubated cells than in non-incubated cells, are identified as having cancer prophylaxis effects with less risk of side effects.

Another embodiment of the method of determining compounds effective in cancer prophylaxis in an individual is described in claim 23.

The cells removed before and after administration are exposed to a mutagenic stimulant in vitro, levels of the growth-suppressing marker are measured in the exposed cells and in the unexposed cells removed before administration, and the cancer prophylaxis effects of the compound are determined based on the post-exposure difference in levels of the growth-suppressing marker, in the cells removed before and after administration. In an aspect of this embodiment, the levels of the growth-suppressing marker are measured in cells removed after administration that are not exposed to a mutagenic stimulant.

The cells are derived from whole blood of the individual. In a further aspect of this embodiment, the levels measured are mRNA levels. In a further aspect of this embodiment, the individual is a human. The growth-suppressing marker is the cytostatic marker p21, the cytocidal marker BAX, and/or the cytocidal marker PUMA.

In a further aspect of this embodiment, the mutagenic stimulant is ionizing radiation. In a further aspect of this embodiment, the level of a plurality of growth-suppressing markers is measured. In a further aspect of this embodiment, the plurality of growth-suppressing markers includes at least one cytostatic marker and one cytocidal marker. In a further aspect of this embodiment, the cytostatic marker is p21 and the cytocidal marker is PUMA.

In a further aspect of this embodiment, compounds exhibiting a greater increase in post-exposure levels of the growth-suppressing marker in cells removed after administration than in cells removed before administration are identified as having cancer prophylaxis effects. In a further aspect of this embodiment, compounds exhibiting no or a small increase in levels of the growth-suppressing marker in post-administration unexposed cells relative to pre-administration unexposed cells, and exhibiting a greater increase in post-exposure levels of the growth-suppressing marker in cells removed after administration than in cells removed before administration, are identified as having cancer prophylaxis effects with less risk of side effects.

### BRIEF DESCRIPTION OF THE DRAWINGS

Figure 1 shows radiation-induced p21 induction in heparinized whole blood.
Figure 2 shows radiation-induced p21 induction in healthy adults (●) and a cancer patient (○).
Figure 3 shows radiation-induced BAX induction in healthy adults (●) and a cancer patient (○).
Figure 4 shows radiation-induced p21 and BAX induction in healthy adults (●,◆,▲) and a cancer patient (○).
Figure 5 shows the attenuation of radiation-induced p21 by various dietary supplements.

### DETAILED DESCRIPTION OF THE PREFERRED EMBODIMENT

Without wishing to be bound by any particular theory, the present inventor believes that the risk of developing cancer can result from an altered response to DNA damage. Moreover, improvements in response to DNA damage can indicate an increased ability to defend against cancer. Thus, one aspect of the present invention relates to a method of measuring cancer susceptibility and of assessing cancer prophylaxis effects of compounds in a particular individual based on the effects of DNA damage on the individual's cells. One embodiment of this method depends on the measurement of the level of a marker mRNA in cells such as whole blood cells. A method for accomplishing measurement of mRNA levels suitable for this purpose was disclosed in United States Patent Application Pub. No. 2004/0265864. The mRNA measurement method employed in the present method may be summarized as follows.

### Measurement of mRNA in Whole Blood

The mRNA measurement protocol employed in the disclosed method allows analysis of larger volumes of unprepared whole blood, provides an efficient means of analyzing mRNA that is derived exclusively from white blood cells; removes rRNA and tRNA, provides consistent mRNA recovery, and is easily adaptable to automation. A sensitive quantification system is provided, including: absolute quantification using real time PCR, and excellent reproducibility with coefficients of variation ranging from 20-25%.

The assay procedure consists of 3 major steps: 1) leukocyte isolation and lysis on filterplates, 2) mRNA isolation, reverse primer hybridization, and cDNA synthesis in oligo(dT)-immobilized microplates, and 3) real time quantitative PCR. In brief, filterplates are placed over collection plates, and 150 µL 5 mmol/L Tris, pH 7.4, is applied to wet the filter membranes. Following centrifugation at 120 xg for 1 min at 4°C to remove solution from filterplates, 50 µL of well-mixed blood samples are applied to each well and immediately centrifuged at 120 xg for 2 min at 4°C, followed by washing of each well with 300 µL phosphate buffered saline (PBS) once with centrifugation at 2000 xg for 5 min at 4°C. Then, 60 µL stock lysis buffer, supplemented with 1% 2-mercaptoethanol (Bio Rad), 0.5 mg/mL proteinase K (Pierce), 0.1 mg/mL salmon sperm DNA (5 Prime Eppendorf/Brinkmann), 0.1 mg/mL *E. coli* tRNA (Sigma), 10 mmol/L each of specific reverse primers, and 10⁷ molecules/mL of synthetic RNA34 as an internal standard, are applied to the filterplates, followed by incubation at 37°C for 10 min. The filterplates are then placed over oligo(dT)-immobilized microplates (GenePlate, RNAture), and centrifuged at 2000 xg for 5 min at 4°C. Following overnight storage at 4°C, the microplates are washed with 100 µL plain lysis buffer 3 times, and then with 150 µL wash buffer (0.5 mol/L NaCl, 10 mmol/L Tris, pH 7.4, 1 mmol/L EDTA) 3 times at 4°C. The cDNA is directly synthesized in each well by adding 30 µL buffer containing 1x RT-buffer (50 mmol/L KCl, 10 mmol/L Tris-HCl, pH 8.3, 5.5 mmol/L MgCl₂, no dithiothreitol), 1.25 mmol/L each of dNTP, 4 units rRNasin, and 80 units of MMLV reverse transcriptase (Promega) (without primers), and incubation at 37°C for 2 hours. The resultant 4 µL cDNA is directly transferred to 384-well PCR plates, to which 5 µL TaqMan universal master mix (Applied Biosystems) and 1 µL oligonucleotide cocktail (15 µmol/L each of forward and reverse primer, and 3-6 µmol/L TaqMan probe) are applied, and PCR is conducted in a PRISM 7900HT (Applied Biosystems), with one cycle of 95°C for 10 min followed by 45 cycles of 95°C for 30 sec, 55°C for 30 sec, and 60°C for 1 min. Each gene is amplified in separate wells. The cycle threshold (Ct), which is the cycle of PCR to generate certain amounts of PCR products (fluorescence), is determined using analytical software (SDS, Applied Biosystems). PCR may be conducted directly in the GenePlate using an iCycler (BioRad).

A simple, reproducible, and high throughput method of mRNA quantification from whole blood is employed. The rapid protocol minimizes the secondary induction or degradation of mRNA after blood draw, and the use of 96-well filterplates and microplates allows the simultaneous manipulation of 96 samples. Minimal manipulation during the procedure provides for very small sample-to-sample variation, with coefficient of variation (CV) values of less than 30%, even when PCR is used as a means of quantification.

In this embodiment, the filter plate may be prepared as follows. Either glassfiber membranes or leukocyte filter membranes can be used to capture leukocytes. In order to simplify the assay, multiple-well filterplates are constructed using glassfiber membranes or leukocyte filter membranes to enable the simultaneous processing of multiple blood specimens. Examples of filters for capturing leukocytes are disclosed in U.S. Patent Nos. 4,925,572 and 4,880,548. These references generally disclose devices for the depletion of leukocytes in blood products or platelet concentrates. The device disclosed in U.S. Patent No. 4,925,572 for use with blood products comprises: an upstream porous element including means for removal of gels such as a needled fibrous non-woven web, at least one intermediate porous element including means for removal of microaggregates such as two or three layers of melt-blown web, and a downstream element including means for removal of leukocytes by both adsorption and filtration such as a number of layers of relatively smaller diameter fibrous web, preferably with at least one of the elements having been modified to a critical wetting surface tension in excess of 0.053N/m (53 dynes/cm). The device disclosed in U.S. Patent No. 4,880,548 for use with platelet concentrate comprises a modified porous, fibrous medium with a critical wetting surface tension of at least about 0.090N/m (90 dynes/cm). That patent also discloses a method for the depletion of the leukocyte content and platelet concentrate comprising passing the platelet concentrate through the porous medium.

Adsorption of leukocytes on fiber surfaces is generally accepted as the mechanism of leukocyte removal. Since the surface area of a given weight of fibers is inversely proportional to the diameter of the fibers, it is to be expected that finer fibers will have higher capacity and that the quantity as measured by weight of fibers necessary to achieve a desired efficiently will be less if the fibers used are smaller in diameter. A number of commonly used fibers, including polyesters, polyamides, and acrylics, lend themselves to radiation grafting, as they have adequate resistance to degradation by γ-radiation at the levels required for grafting and are of a structure with which available monomers can react. PBT has been the principal resin used for the development of the products of this invention and is the resin used in the examples. It should be noted, however, that other resins may be found which can be fiberized and collected as mats or webs with fibers as small as 1.5 micrometers or less, and that such products, with their critical wetting surface tensions adjusted as necessary to the optimum range, may be well suited to the fabrication of equally efficient but still smaller leukocyte depletion devices. Similarly, glass fibers, appropriately treated, may be usable to make effective devices. Absorption of CD4 mRNA is up to four times as effective when using PBT-based filters as opposed to glass fiber-based filters. In one preferred embodiment, multiple filter membranes are layered together to increase the amount of leukocytes captured from whole blood. In another preferred embodiment, the filter plate is sealed with a plastic adhesive tape (Bio-Rad 223-9444), and the tape is cut to allow access to a desired number of wells. In another preferred embodiment, each well to which a sample will be added is washed with a hypotonic buffer (200 µL 5 mM Tris, pH 7.4).

The method preferably involves collecting blood, adding the blood to the multi-well filter plate, and removal of erythrocytes and other non-leukocyte components. In one preferred embodiment, whole blood can be drawn into blood collection tubes containing anticoagulants, which increase the efficiency of the leukocyte filtering. The anticoagulant heparin is particularly effective in increasing the efficiency of leukocyte filtering. Other anticoagulants such as ACD and EDTA may also be employed, but the signal strength in the resulting mRNA measurement may be reduced. In one preferred embodiment, the blood sample can be frozen, which removes some of the RNAases that destroy mRNA. The wells can be washed with a hypotonic buffer. Once blood has been added to the desired number of wells on the filterplate, the blood is filtered through the filter membrane. Filtration can be affected through any technique known to those of skill in the art, such as centrifugation, vacuum aspiration, or positive pressure.

The method involves cell lysis and hybridization of mRNA to the oligo(dT)-immobilized within the mRNA capture zone. Lysis buffer is applied to the filterplate wells (40µL/well), and incubation is allowed to occur (room temperature for 20 min) to release mRNA from the trapped leukocytes. In one preferred embodiment, the multi-well filterplate is sealed in a plastic bag and centrifuged (IEC MultiRF, 2000 rpm, at 4 C, for 1 min). Lysis buffer is then added again (20 µL/well), followed by centrifugation (IEC MultiRF, 3000 rpm, at 4 C, for 5 min). The multi-well filterplate is then removed from the centrifuge and incubated (room temperature for 2 hrs).

In accordance with a preferred embodiment, the lysis buffer comprises a detergent, a salt, a pH buffer, guanidine thiocyanate, and proteinase K.

Preferred embodiments of the lysis buffer contain at least one detergent, but may contain more than one detergent. Those skilled in the art may utilize different combinations of concentrations of detergents with different strengths in order to achieve varying levels of lysis of different membranes for various types of cells. For example, IGEPAL CA-630 is a weaker detergent than N-laurosarcosine, and in one embodiment IGEPAL CA-630 alone may be sufficient to lyse a cytoplasmic membrane. In other embodiments, a strong detergent, such as N-laurosarcosine can be used in combination with one or more weak detergents to optimize lysis of nuclear membranes. The detergents are preferably sufficient to lyse at least the cytoplasmic membrane of cells. Another preferred embodiment comprises a detergent sufficient to lyse the nuclear membrane of cells, as significant amounts of mRNA reside in the nuclei of cells. In some circumstances it is desirable to measure only cytoplasmic mRNA, while in other circumstances, it may be desirable to measure mRNA in the cytoplasm and nucleus.

Strong detergents of the lysis buffer preferably include, but are not limited to: N-lauroylsarcosine, S.D.S., Sodium deoxycholate, and Hexadecyltrimethylammonium bromide.

Weak detergents include IGEPAL CA-630, N-Decanoyl-N-methylglucamine, Octyl-β-D-glucopyranoside, or other detergents known to those skilled in the art. 0.05-2% detergent can be used in the lysis buffer. One particularly preferred embodiment of the lysis buffer includes 0.5% N-lauroylsarcosine. Another preferred embodiment of the lysis buffer contains 0.1-2% IGEPAL CA-630. A particularly preferred embodiment contains 0.1% IGEPAL CA-630.

The combination of salts and chelating agents can also serve as a lysing agent. For example, 75 µM NaCl and 24 µM Na-EDTA can serve as a lysing agent. Embodiments of lysing agents may include other lysing agents known to those skilled in the art.

The salt of the lysis buffer acts as an mRNA-oligo(dT) hybridizing agent. The salt should preferably have a stringency (the rigor with which complementary DNA sequences hybridize together) that does not exceed that of 4X SSC, as determinable by those skilled in the art. Other embodiments of the lysis buffer include NaCl or other salts known to those skilled in the art.

The pH buffer of the lysis buffer stock preferably maintains a pH of 7.0-8.0. One embodiment comprises 1 mM-100 mM Tris HCl, pH 7.4. In a particularly preferred embodiment, the pH buffer comprises 10 mM Tris HCl, pH 7.4. Other preferred embodiments of the lysis buffer include pH buffers known to those skilled in the art, including 0.1 M Citrate-Phosphate, pH 5.0, with 0.03% H₂O₂.

In accordance with a particularly preferred embodiment of the lysis buffer, guanidine thiocyanate serves as an RNAase deactivating agent. We have discovered that guanidine thiocyanate has typically been used in the prior art at insufficient concentrations to be effective. Therefore, preferably, the concentration of guanidine thiocyanate is greater than 1.4 M. Guanidine thiocyanate concentration as high as 10 M, more preferably no higher than 2 M can be used. However, at concentrations above 1.7 M, the efficiency of the lysis buffer is decreased. Accordingly, the preferred embodiment uses about 1.4 to about 1.75 M guanidine thiocyanate. One preferred embodiment comprises 1.7-1.8 M guanidine thiocyanate. A working lysis buffer can be prepared from a stock to achieve a particular concentration of 1.791 M guanidine thiocyanate. As other reagents are added to the lysis buffer, the concentration of guanidine thiocyanate becomes diluted. The lysis buffer preferably comprises guanidine thiocyanate in concentrations of about 1.6 to about 1.7 M.

A particularly preferred embodiment further comprises 20 mg/ml of proteinase K as an RNAase inactivating agent. One preferred embodiment of the lysis buffer comprises 200 µg/ml-20 mg/ml of proteinase K. Another preferred embodiment comprises 200 µg/ml -1.0 mg/ml proteinase K. Another preferred embodiment comprises 200 µg/ml -500µg/ml proteinase K. Sodium dodecyl sulfate may also serve as the RNAase deactivating agent. Another embodiment includes 0.1-10% of 2-mercaptoethanol as an RNAase inactivating agent. One particularly preferred embodiment comprises 1% 2-mercaptoethanol. Other embodiments of RNAase inactivating agents may preferably include materials, known to those skilled in the art, that reduce disulfide bonds in RNAases.

Preferred embodiments of the lysis buffer further comprise chelating agents which chelate Mg²⁺ and Ca²⁺. One preferred embodiment comprises 0.1 mM-5 mM EDTA. A particularly preferred embodiment comprises 1 mM EDTA. Other preferred embodiments of the lysis buffer stock contain chelating agents known to those skilled in the art including, for example and without limitation, EDTMP, 2,3-dimercaptopropanol, and EGTA.

Preferred embodiments of the lysis buffer may include tRNA, which may come from various sources and is included in order to inhibit non-specific absorption of blood-derived DNA and RNA to filter plates. Additionally, the presence of tRNA prevents degradation of blood-derived RNA. In one preferred embodiment, the tRNA of the working lysis buffer comprises 0.1-10 mg/ml of E. coli tRNA. Other embodiments may contain tRNA from any source known to those skilled in the art.

Preferred embodiments of the lysis buffer may include DNA from a wide variety of sources, which is added in order to inhibit non-specific absorption of blood-derived DNA and RNA to filter plates. The DNA of the working lysis buffer preferably comprises 0.1-10 mg/ml of sonicated salmon sperm DNA. In other embodiments, DNA from other organisms may be used.

Particularly preferred embodiments of the lysis buffer may include spiked control RNA to calculate the definite quantity of target mRNAs in the original samples. Prior to embodiments of the present invention, it was difficult to compare the results in one experiment to those in other experiments due to institute-to-institute variation and lack of standardization. However, in preferred embodiments of the present invention a definite quantity of target mRNA can be determined by dividing the values obtained by the TaqMan or other PCR assay with percent recovery of a dose of spiked control RNA in each sample.

Preferred embodiments of the lysis buffer include 10 to 1e¹⁰, more preferably 1e⁵ to 1e¹⁰, copies of spiked RNA per well. In preferred embodiments, the amount of control RNA used is at least enough to be detected, but not so much as to significantly interfere with the amount of target mRNA that is quantified. In preferred embodiments, the control RNA added to the lysis buffer is poly(A)⁺ RNA. In particularly preferred embodiments where the sample being tested is human blood, the control RNA is not homologous to RNA present in human blood. In some preferred embodiments, the sequence of the control RNA is less than 90% homologous to the target mRNA, or has greater than 10% difference in length with the target mRNA. In other preferred embodiments, the sequence of the control RNA is less than 85% homologous to the target mRNA, or has greater than 5% difference in length with the target mRNA. In further embodiments, the sequence of the control RNA is less than 75% homologous to the target mRNA, or has greater than 2% difference in length with the target mRNA. In alternative embodiments, the sequence of the control RNA is less than 65% homologous to the target mRNA, or has greater than 1% difference in length with the target mRNA. In one embodiment, control RNA may preferably be made by amplifying template oligonucleotides by means of PCR. In one embodiment, control RNA may preferably be made by amplifying template oligonucleotides by means of PCR. Thus, forward primers (SEQ ID NOs 3, 4, 7, and 1), reverse primers (SEQ ID NOs 2 and 8), and TaqMan probes (FAM-SEQ ID NO 6-TAMRA, FAM-SEQ ID NO 9-TAMRA, and FAM-SEQ ID NO 5-TAMRA) can be used to amplify various control RNA oligonucleotides. Alternative embodiments comprise using a plurality of different target mRNAs to be quantified. Further embodiments comprise using a plurality of control RNAs.

The method involves quantification of mRNA, which in a preferred embodiment entails cDNA synthesis from mRNA and amplification of cDNA using PCR. In one preferred embodiment, the multi-well filterplate is washed with lysis buffer (150 µL/well x 3 times, manual) and wash buffer (150 µL/well x 3 times, manual or BioTek #G4). A cDNA synthesis buffer is then added to the multi-well filterplate (40 µL/well, manual or I&J #6). Axymat (Amgen AM-96-PCR-RD) can be placed on the multi-well filterplate, which is then placed on a heat block (37°C, VWR) and incubated (>90 min). The multi-well filterplate can then be centrifuged (2000 rpm, at 4°C for 1 min). PCR primers are added to a 384 well PCR plate, and the cDNA is transferred from the multi-well filterplate to the 384 well PCR plate. The PCR plate is centrifuged (2000 rpm, at 4°C for 1 min), and real time PCR is commenced (TaqMan/SYBER).

Disclosed is the application of specific antisense primers during mRNA hybridization or during cDNA synthesis. It is preferable that the primers be added during mRNA hybridization, so that excess antisense primers may be removed before cDNA synthesis to avoid carryover effects. The oligo(dT) and the specific primer (NNNN) simultaneously prime cDNA synthesis at different locations on the poly-A RNA. The specific primer (NNNN) and oligo(dT) cause the formation of cDNA during amplification. Even when the specific primer-derived cDNA is removed from the GenePlate by heating each well at 95 degrees C for two minutes, the amounts of specific CD4 cDNA obtained from the heat denaturing process (using TaqMan quantitative PCR) is similar to the amount obtained from an un-heated negative control. Without wishing to be bound by any explanation or theory, one possible explanation for such results is that oligo(dT)-derived cDNA may displace primer-derived cDNA during amplification. This is particularly convenient because the heat denaturing process is completely eliminated. Moreover, by adding multiple antisense primers for different targets, each gene can be amplified from the aliquot of cDNA, and oligo(dT)-derived cDNA in the GenePlate can be stored for future use.

Further disclosed is a device for high-throughput quantification of mRNA from whole blood. The device includes a multi-well filterplate containing: multiple sample-delivery wells, a leukocyte-capturing filter underneath the sample-delivery wells, and an mRNA capture zone under the filter, which contains oligo(dT)-immobilized in the wells of the mRNA capture zone. In order to increase the efficiency of leukocyte collection, several filtration membranes can be layered together.

Although many conventional amplification techniques can be used in conjunction with this measurement method, one particularly preferred embodiment of the present invention comprises conducting real-time quantitative PCR (TaqMan) with whole blood-derived RNA and control RNA. Holland, et al., PNAS 88:7276-7280 (1991) describe an assay known as a Taqman assay. The 5' to 3' exonuclease activity of Taq polymerase is employed in a polymerase chain reaction product detection system to generate a specific detectable signal concomitantly with amplification. An oligonucleotide probe, nonextendable at the 3' end, labeled at the 5' end, and designed to hybridize within the target sequence, is introduced into the polymerase chain reaction assay. Annealing of the probe to one of the polymerase chain reaction product strands during the course of amplification generates a substrate suitable for exonuclease activity. During amplification, the 5' to 3' exonuclease activity of Taq polymerase degrades the probe into smaller fragments that can be differentiated from undegraded probe. The assay is sensitive and specific and is a significant improvement over more cumbersome detection methods. A version of this assay is also described in Gelfand et al., in U.S. Patent No. 5,210,015. That patent discloses a method comprising: (a) providing to a PCR assay containing a sample, at least one labeled oligonucleotide containing a sequence complementary to a region of the target nucleic acid, wherein the labeled oligonucleotide anneals within the target nucleic acid sequence bounded by the oligonucleotide primers of step (b); (b) providing a set of oligonucleotide primers, wherein a first primer contains a sequence complementary to a region in one strand of the target nucleic acid sequence and primes the synthesis of a complementary DNA strand, and a second primer contains a sequence complementary to a region in a second strand of the target nucleic acid sequence and primes the synthesis of a complementary DNA strand; and wherein each oligonucleotide primer is selected to anneal to its complementary template upstream of any labeled oligonucleotide annealed to the same nucleic acid strand; (c) amplifying the target nucleic acid sequence employing a nucleic acid polymerase having 5' to 3' nuclease activity as a template dependent polymerizing agent under conditions which are permissive for PCR cycling steps of (i) annealing of primers and labeled oligonucleotide to a template nucleic acid sequence contained within the target region, and (ii) extending the primer, wherein said nucleic acid polymerase synthesizes a primer extension product while the 5' to 3' nuclease activity of the nucleic acid polymerase simultaneously releases labeled fragments from the annealed duplexes comprising labeled oligonucleotide and its complementary template nucleic acid sequences, thereby creating detectable labeled fragments; and (d) detecting and/or measuring the release of labeled fragments to determine the presence or absence of target sequence in the sample. U.S. Pat. No. 5,210,015 to Gelfand, et al., and Holland, et al., PNAS 88:7276-7280 (1991).

Further, U.S. Patent No. 5,491,063 to Fisher, et al., provides a Taqman-type assay. The method of Fisher et al. provides a reaction that results in the cleavage of single-stranded oligonucleotide probes labeled with a light-emitting label wherein the reaction is carried out in the presence of a DNA binding compound that interacts with the label to modify the light emission of the label. The method utilizes the change in light emission of the labeled probe that results from degradation of the probe. The methods are applicable in general to assays that utilize a reaction that results in cleavage of oligonucleotide probes, and in particular, to homogeneous amplification/detection assays where hybridized probe is cleaved concomitant with primer extension. A homogeneous amplification/detection assay is provided which allows the simultaneous detection of the accumulation of amplified target and the sequence-specific detection of the target sequence, see U.S. Patent No. 5,491,063 to Fisher, et al.

The TaqMan detection assays offer several advantages over the classical PCR assays. First, the TaqMan assays combine the sensitivity of PCR along with hybridization of the internal oligonucleotide sequence that is present in a target sequence. Following PCR, samples do not have to be separated on agarose gels, and the subsequent Southern blots and hybridization steps that are necessary to verify the identity of the PCR products is eliminated. These additional post-PCR confirmation steps can easily add several days for an accurate identification. Using the TaqMan system, the assays are completed within 2.5 h. Further, the methodology involved in the assay process makes possible the handling of large numbers of samples efficiently and without cross-contamination and is therefore adaptable for robotic sampling. As a result, large numbers of test samples can be processed in a very short period of time using the TaqMan assay. Another advantage of the TaqMan system is the potential for multiplexing. Since different fluorescent reporter dyes can be used to construct probes, several different HIV systems could be combined in the same PCR reaction, thereby reducing the labor costs that would be incurred if each of the tests were performed individually. The advantages of rapid, conclusive data together with labor and cost efficiency make the TaqMan detection system utilizing the specific primers of the invention a highly beneficial system for monitoring the presence of HIV.

Other real-time PCR formats may also be employed. One format employs an intercalating dye, such as SYBR Green. This dye provides a strong fluorescent signal on binding double-stranded DNA; this signal enables quantification of the amplified DNA. Although this format does not permit sequence-specific monitoring of amplification, it enables direct quantization of amplified DNA without any labeled probes (see, e.g., Ponchel et al. (2003) Real-time PCR based on SYBR-Green I fluorescence: An alternative to the TaqMan assay for a relative quantification of gene rearrangements, gene amplifications and micro gene deletions. BMC Biotechnology 3:18). Other such fluorescent dyes that may also be employed are SYBR Gold, YO-PRO dyes and Yo Yo dyes.

Another real-time PCR format that may be employed uses reporter probes that hybridize to amplicons to generate a fluorescent signal. The hybridization events either separate the reporter and quencher moieties on the probes or bring them into closer proximity. The probes themselves are not degraded and the reporter fluorescent signal itself is not accumulated in the reaction. The accumulation of products during PCR is monitored by an increase in reporter fluorescent signal when probes hybridize to amplicons. Formats in this category include molecular beacons (Sanjay, T and Russell, K (1996) Molecular Beacons: Probes that Fluoresce upon Hybridization. Nature Biotech. Vol. 14, March, pp303-308), dual-hybe probes (Cardullo et al. (1988) Detection of nucleic acid hybridization by nonradiative fluorescence resonance energy transfer. PNAS USA 85:8790-8794), Sunrise or Amplifluor (Nazarenko, et al. (1997) A closed tube format for amplification and detection of DNA based on energy transfer. Nucleic Acid Res. 25:2516-2521), and Scorpion (Whitcombe et al. (1999) Detection of PCR products using self quenching probing amplicons and fluorescence. Nature Biotech. 17:804-807) real-time PCR assays.

Another real-time PCR format that may also be employed is the so-called "Policeman" system. In this system, the primer comprises a fluorescent moiety, such as FAM, and a quencher moiety which is capable of quenching fluorescence of the fluorescent moiety, such as TAMRA, which is covalently bound to at least one nucleotide base at the 3' end of the primer. At the 3' end, the primer has at least one mismatched base and thus does not complement the nucleic acid sample at that base or bases. The template nucleic acid sequence is amplified by PCR with a polymerase having 3'-5' exonuclease activity, such as the Pfu enzyme, to produce a PCR product. The mismatched base(s) bound to the quencher moiety are cleaved from the 3' end of the PCR product by 3'-5' exonuclease activity. The fluorescence that results when the mismatched base with the covalently bound quencher moiety is cleaved by the polymerase, thus removing the quenching effect on the fluorescent moiety, is detected and/or quantified at at least one time point during PCR. Fluorescence above background indicates the presence of the synthesized nucleic acid sample.

In preferred embodiments, various mRNAs can be quantitated by simply changing primers and probes for each target. Because heparin maintains extracellular Ca⁺⁺, which is an important factor for maximum biological activity, drug actions can be analyzed in whole blood without isolating leukocytes.

The ability to determine the total efficiency of a given sample by using known amounts of spiked standard RNA results from embodiments being dose-independent and sequence-independent. The use of known amounts of control RNA allows PCR measurements to be converted into the quantity of target mRNAs in the original samples.

Disclosed is a kit for high-throughput quantification of mRNA from whole blood. The kit includes: the device for high-throughput quantification of mRNA from whole blood; heparin-containing blood-collection tubes; a hypotonic buffer; and a lysis buffer.

Further disclosed is a fully automated system for performing high throughput quantification of mRNA in whole blood, including: robots to apply blood samples, hypotonic buffer, and lysis buffer to the device; an automated vacuum aspirator and centrifuge, and automated PCR machinery.

The method of measuring cancer susceptibility disclosed may also employ other methods of measuring mRNA other than those described above. Other methods which may be employed include, for example, Northern blot analysis, Rnase protection, solution hybridization methods, semi-quantitative RT-PCR, and in situ hybridization.

### Measurement of Cancer Susceptibility

The present method measures the levels of a growth-suppressing marker as defined in the claims in whole blood of an individual. In a preferred embodiment, the marker levels measured are mRNA levels. However, the level of the marker may also be assessed in other ways known to those of skill in the art, such as by determining levels of the marker protein present in the cells of the individual. As used in the present application, an "individual" may be an animal of any species, including a human being. Furthermore, as used in the present application, the "growth-suppressing" mRNAs include both cytocidal mRNAs, which stop cell proliferation, and cytocidal mRNAs that kill cells. In the following examples, a larger increase in the level of growth-suppressing mRNAs after mutagenic stimulus indicates a lower risk of cancer, while a decrease indicates a higher risk of cancer.

Using the present method, the measurement of an individual's susceptibility to cancer is correlated to the increase in the cytostatic marker mRNA that results from exposure to a mutagenic stimulant such as ionizing radiation. The greater the increase in the marker mRNA in response to the stimulus, the less susceptible the individual will be to the accumulation of DNA damage that can result in cancer. If an individual exhibits little or no marker mRNA induction in response to exposure to a mutagenic stimulus, however, it is likely that DNA damage will accumulate in proliferating cells, eventually resulting in the development of cancer.

Although the examples below employ the preferred protocol of measuring the change in marker mRNA levels in an individual directly before and after exposure to mutagenic stimulus, it is also possible to obtain an average baseline measurement of the level of the marker mRNA in unexposed cells from a number of individuals of the same species, and compare the post-exposure marker mRNA level in the individual's cells to the average baseline measurement to determine the change in the marker mRNA levels. This has the advantage of only requiring one mRNA level measurement to determine the individual's cancer susceptibility. The average baseline measurement is preferably obtained based on marker mRNA measurements from at least 10 individuals. It is more preferably obtained based on marker mRNA measurements from at least 25 individuals. It is most preferably obtained based on marker mRNA measurements from at least 50 individuals.

A wide variety of mutagenic stimulants can be employed in the method of the present invention. Such mutagenic stimulants may include, for example, physical agents such as heat, UV radiation, or ionizing radiation such as X-rays or gamma rays. The mutagenic stimulant may also be a chemical agent, including base analogs such as bromouracil and aminopurine; agents that alter base structure and pairing properties such as nitrous acid, nitrosoguanidine, ethyl methanesulphonate, and methyl methanesulphonate; intercalating agents such as acridine orange, proflavin, and ethidium bromide; structure-altering agents such as NAAAF, psoralens, and peroxides; and anti-tumor drugs such as bleomycin and etoposide (VP-16). The use of transposable elements such as retroviruses is also contemplated.

Several cytostatic markers are known, such as p21, p27^{Kip1} and p16/p15^{INK4}. Since p21 is well known to stop cell proliferation in response to DNA damage, p21 was used in the examples as a cytostatic marker for radiation-induced cell arrest.

Similarly, in the exmples, the cytocidal markers BAX and PUMA, which are known to have strong pro-apoptotic effects, were measured. It was determined by the present inventor that PUMA is the dominant pro-apoptotic mRNA in human blood.

### Example 1

The protocol for measuring mRNA levels employed in this example was as follows. The assay procedure consists of 3 major steps: 1) leukocyte isolation and lysis on filterplates, 2) mRNA isolation, reverse primer hybridization, and cDNA synthesis in oligo(dT)-immobilized microplates, and 3) real time quantitative PCR. In brief, filterplates were placed over collection plates, and 150 µL 5 mmol/L Tris, pH 7.4, was applied to wet the filter membranes. Following centrifugation at 120 xg for 1 min at 4°C to remove solution from the filterplates, 50 µL of well-mixed blood samples were applied to each well and immediately centrifuged at 120 xg for 2 min at 4°C, followed by washing of each well. with 300 µL phosphate buffered saline (PBS) once with centrifugation at 2000 xg for 5 min at 4°C. Then, 60 µL stock lysis buffer (see below), supplemented with 1% 2-mercaptoethanol (Bio Rad), 0.5 mg/mL proteinase K (Pierce), 0.1 mg/mL salmon sperm DNA (5 Prime Eppendorf/Brinkmann), 0.1 mg/mL *E. coli* tRNA (Sigma), 10 mmol/L each of specific reverse primers, and 10⁷ molecules/mL of synthetic RNA34 as an internal standard, were applied to the filterplates, followed by incubation at 37°C for 10 min. The filterplates were then placed over oligo(dT)-immobilized microplates (GenePlate, RNAture), and centrifuged at 2000 xg for 5 min at 4°C. Following overnight storage at 4°C, the microplates were washed with 100 µL plain lysis buffer 3 times, and then with 150 µL wash buffer (0.5 mol/L NaCl, 10 mmol/L Tris, pH 7.4, 1 mmol/L EDTA) 3 times at 4°C. The cDNA was directly synthesized in each well by adding 30 µL buffer containing 1x RT-buffer (50 mmol/L KCl, 10 mmol/L Tris-HCl, pH 8.3, 5.5 mmol/L MgCl₂, no dithiothreitol), 1.25 mmol/L each of dNTP, 4 units rRNasin, and 80 units of MMLV reverse transcriptase (Promega) (without primers), and incubation at 37°C for 2 hours. The resultant 4 µL cDNA was directly transferred to 384-well PCR plates, to which 5 µL TaqMan universal master mix (Applied Biosystems) and 1 µL oligonucleotide cocktail (15 µmol/L each of forward and reverse primer, and 3-6 µmol/L TaqMan probe) were applied, and PCR was conducted in a PRISM 7900HT (Applied Biosystems), with one cycle of 95°C for 10 min followed by 45 cycles of 95°C for 30 sec, 55°C for 30 sec, and 60°C for 1 min. Each gene was amplified in separate wells. The cycle threshold (Ct), which was the cycle of PCR to generate certain amounts of PCR products (fluorescence), was determined using analytical software (SDS, Applied Biosystems). PCR was also conducted directly in the GenePlate using an iCycler (BioRad).

### Lysis Buffer stock

0.5% N-Lauroylsarcosine
4X SSC
10 mM Tris HCl, pH 7.4
1 mM EDTA
0.1% IGEPAL CA-630
1.791 M guanidine thiocyanate

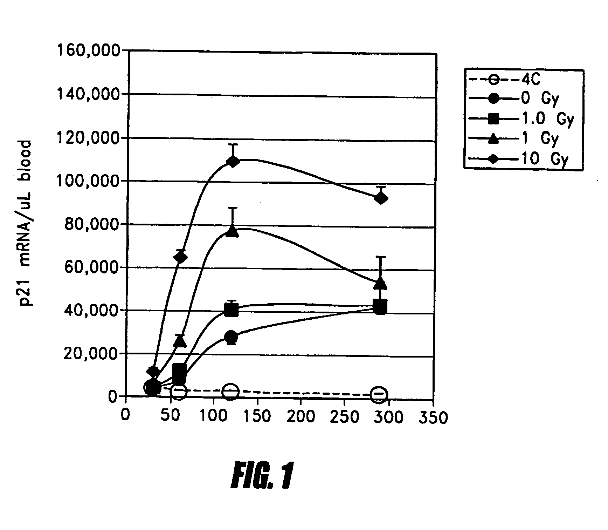

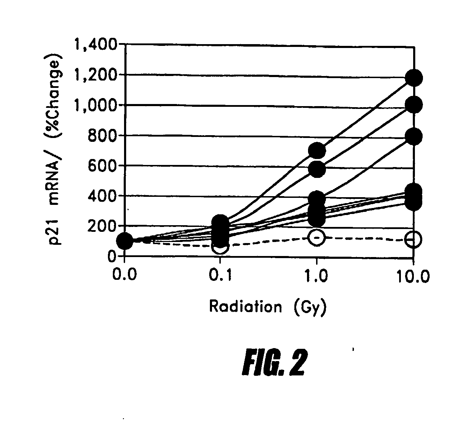

The measurement of p21 mRNA was conducted both before and after exposure to varying doses of ionizing radiation. The results are shown in Figure 1. The results demonstrate that p21 mRNA was induced in a dose dependent manner when heparinized whole blood was exposed to ionizing radiation in vitro. The expression was maximal as early as 2 hours after radiation exposure. Furthermore, as shown in Figure 2, when a cancer patient who developed 2 independent malignant cancers and 1 independent benign tumor was tested using the same protocol, the induction of p21 mRNA was very low (open circles).

### Example 2

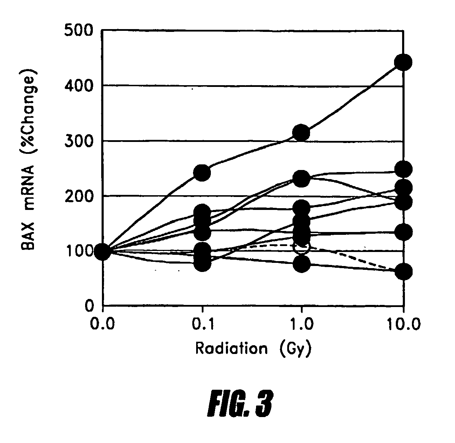

Using the same protocol as above, the mRNA levels of the cytocidal marker BAX were measured. BAX is considered to be a cytocidal marker because BAX mRNA is induced at the early stages of apoptosis. As shown in Figure 3, the cancer patient of Example 1 exhibited a decrease in BAX mRNA upon stimulation with 10 Gy of radiation, whereas the majority of healthy adults tested exhibited an increase in BAX mRNA after radiation stimulus.

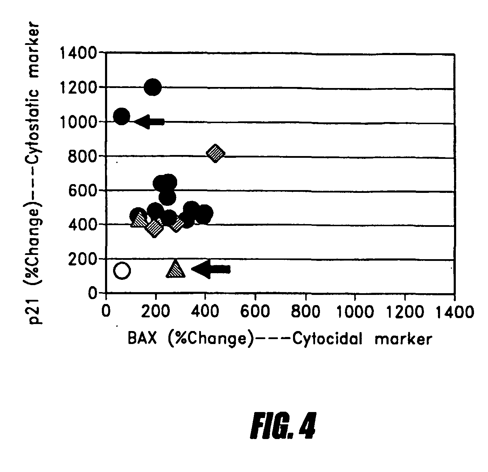

When Fig. 2 and Fig. 3 were combined, and more healthy adults' data were added (Fig. 4), the cancer patient exhibited poor responses for both p21 and BAX. In Figure 4, 4 data points (◆) and 2 data points (▲) were derived from the same individuals tested at different dates. One healthy adult exhibited a poor BAX response, but this was counterbalanced by an elevated p21 response (Figure 4,← ). Similarly, one healthy adult who exhibited a poor p21 response exhibited a compensating elevated BAX response (Fig. 4, ←). Thus, cancer susceptibility or cancer risk may be identified by the responses of both cytostatic (p21) and cytocidal (BAX) marker genes.

### Example 3

Using the same protocol as above, the mRNA levels of the cytocidal marker PUMA were measured. In human blood, PUMA mRNA has been found to have the strongest pro-apoptotic effects of the BAX family of genes. The blood of a cancer patient stimulated with 10 Gy of radiation exhibited a poor PUMA response, while the majority of healthy adults tested exhibited an increase in PUMA mRNA after radiation stimulus.

### Method of Assaying Compounds for Cancer Prophylaxis Effects in Individuals

Interestingly, the values of Figure 4 were not fixed within the same individuals (◆,▲), and fluctuated over time. This indicates that the cancer risk (DNA damage responses) can be modified. Thus, this test may be applicable to identify cancer preventive regimens for each individual in vivo, or by incubating whole blood with candidate compounds in vitro.

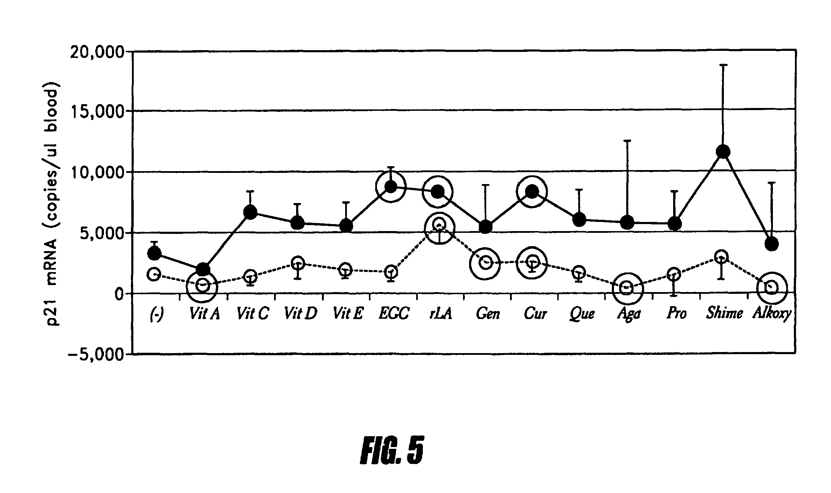

Figure 5 shows the results of an individualized drug screening for cancer prevention. First, heparinized whole blood was incubated with various dietary supplements at 37°C for 1 hour (2 tubes for each compound). Then one tube was exposed to 1 Gy of ionizing radiation, then both tubes were incubated for another 2 hours at 37°C. The p21 mRNA was quantitated as described above. As shown in Figure 5, the leftmost set of data points was obtained without any supplements (labeled (-)) and confirmed radiation-induced p21 induction, which was similar to the results of Fig. 1-3. In Figure 5, each data point is the mean ± standard deviation of p21 mRNA with (●) or without (○) 1 Gy radiation. Dietary supplements are: vitamin A (10 µM), C (10 µg/mL), D (100 nM), and E (1:1000), epigallocatechin (EGC, green tea extract) (10 µM), γ-linoleic acid (rLA) (10 µg/mL), genistein (Gen, soy extract) (10 µM), curcumin (Cur, spice) (1 µM), quecetin (Que, vegetable flavonoid) (100 nM), Agaricus (Aga, mushroom extract) (1:100), propolis (Pro, bee nest extract) (1:1000), shimemakobu (Shi, mushroom extract) (sup of 30 mg/mL), and alkoxy glycerol (Alkoxy, shark extract) (1:100), respectively. Data points surrounded by a circle indicate p<0.05.

Interestingly, some dietary supplements, such as epigallocatechin gallate (green tea extract) significantly enhanced radiation-induced p21 expression, while it did not show any changes in p21 levels on control blood (no radiation). γ-linoleic acid and curcumin (spice) also enhanced radiation-induced p21 expression, although these compounds also increased background p21 levels. The increases in background mRNA expression may indicate some side effect or toxicity. Compounds that exhibit more than a small increase in background marker mRNA expression may have such side effects. As used herein, a "small" or "weak" increase in mRNA expression is preferably less than a 400% increase in expression levels, more preferably less than a 200% increase in expression levels, still more preferably less than a 100% increase in expression levels, and most preferably less than a 50% increase in expression levels. A greater increase is determined to be a "strong" increase. These data indicate that some of these dietary supplements increased DNA damage responses, which may indicate cancer prevention.

It is also possible to screen these compounds in vivo by administering them to an individual before measuring the growth-suppressing marker as described above. The administration may be carried out in any manner known to those of skill in the art, such as orally or intravenously. Appropriate dosages vary depending on the compound administered and may be determined in accordance with standard dosing regimens known to those of skill in the art.

Those compounds above that are extracts may be further tested to identify the active components thereof. This is expected to enhance the cancer prophylaxis effect and may reduce any side effects or toxicity.

As shown by the results of Figure 5, which were derived from a single individual, it is possible to identify suitable compounds for an individual to enhance that individual's ability to defend against the development of cancer. In other words, it is possible to develop a cancer prophylaxis protocol tailored to that individual.

Furthermore, if certain compounds show similar results in many other individuals, these compounds may be assumed to have general cancer prophylaxis effects and be used by the general public for reducing the risk of cancer.

## Claims

1. A method of determining susceptibility to cancer in an individual, comprising:
exposing whole blood of the individual to a mutagenic stimulant in vitro;
measuring the level of the growth-suppressing marker in cells derived from the exposed whole blood and in cells derived from non-exposed whole blood of the individual; and
determining the individual's susceptibility to cancer based on the difference in marker levels in the exposed and non-exposed whole blood, wherein the susceptibility to cancer is determined as being increased when the level of the growth-suppressing marker does not increase or weakly increases after exposure, wherein the growth-suppressing marker is selected from p21, PUMA and BAX.

2. The method of claim 1, wherein the levels measured are mRNA levels.

3. The method of claim 1, wherein the individual is a human.

4. The method of claim 1, wherein the mutagenic stimulant is ionizing radiation.

5. The method of claim 1, wherein the susceptibility to cancer is determined as being decreased when the level of the growth-suppressing marker strongly increases after exposure.

6. The method of claim 1, wherein the level of a plurality of growth-suppressing markers is measured.

7. The method of claim 6, wherein the level of p21 and PUMA is measured.

8. A method of determining susceptibility to cancer in an individual, comprising:
obtaining a baseline average measurement of levels of a growth-suppressing marker in whole blood from a plurality of members of the individual's species after said whole blood had been exposed to a mutagenic stimulant in vitro;
exposing whole blood of the individual to a mutagenic stimulant in vitro;
measuring the level of the growth-suppressing marker in cells derived from the whole blood after exposure; and
determining the individual's susceptibility to cancer, wherein a higher level than the baseline average measurement indicates a lower risk of cancer and a lower level than the baseline average measurement indicates a higher risk of cancer, wherein the growth-suppressing marker is selected from p21, PUMA and BAX.

9. The method of claim 8, wherein the levels measured are mRNA levels.

10. The method of claim 8, wherein the individual is a human.

11. The method of claim 8, wherein the mutagenic stimulant is ionizing radiation.

12. The method of claim 8, wherein the level of a plurality of growth-suppressing markers is measured.

13. The method of claim 12, wherein the level of p21 and PUMA is measured.

14. An in-vitro method of screening a compound for cancer prophylaxis effects in an individual, comprising:
incubating whole blood samples of the individual with the compound in vitro; j eX exposing the incubated whole blood samples and non-incubated whole blood samples of the individual to a mutagenic stimulant in vitro;
measuring levels of growth-suppressing marker in cells derived from the exposed whole blood samples and in cells derived from non-incubated, non-exposed whole blood of the individual; and
identifying compounds having cancer prophylaxis effects based on differences in levels of the growth-suppressing marker in the incubated whole blood and the non-incubated whole blood after exposure, wherein the growth-suppressing marker is selected from p21, PUMA and BAX.

15. The method of claim 14, wherein the levels of the growth-suppressing marker are further measured in incubated whole blood that is not exposed to a mutagenic stimulant.

16. The method of claim 14, wherein the levels measured are mRNA levels.

17. The method of claim 14, wherein the individual is a human.

18. The method of claim 14, wherein the mutagenic stimulant is ionizing radiation.

19. The method of claim 14, wherein the level of a plurality of growth-suppressing markers is measured.

20. The method of claim 19, wherein the level of p21 and PUMA is measured.

21. The method of claim 14, wherein compounds exhibiting a greater increase in post-exposure levels of the growth-suppressing marker in incubated whole blood than in non-incubated whole blood are identified as having cancer prophylaxis effects.

22. The method of claim 15, wherein compounds exhibiting no or a small increase in levels of the growth-suppressing marker in incubated unexposed whole blood relative to unincubated, unexposed whole blood, and exhibiting a greater increase in post-exposure levels of the growth-suppressing marker in incubated whole blood than in non-incubated whole blood, are identified as having cancer prophylaxis effects with less risk of side effects.

23. An in-vitro method of determining whether a compound is effective in cancer prophylaxis in an individual, comprising:
exposing the whole blood samples, which have been removed from the individual before and after administration of the compound, to a mutagenic stimulant in vitro;
measuring levels of a growth-suppressing marker in the cells derived from exposed whole blood samples and in cells derived from unexposed whole blood removed before administration; and
determining the cancer prophylaxis effects of the compound based on the post-exposure difference in levels of the growth-suppressing marker, in the whole blood samples removed before and after administration, wherein the growth-suppressing marker is selected from p21, PUMA and BAX.

24. The method of claim 23, wherein the levels of the growth-suppressing marker are further measured in whole blood removed after administration that are not exposed to a mutagenic stimulant.

25. The method of claim 23, wherein the levels measured are mRNA levels.

26. The method of claim 23, wherein the individual is a human.

27. The method of claim 23, wherein the mutagenic stimulant is ionizing radiation.

28. The method of claim 23, wherein the level of a plurality of growth-suppressing markers is measured.

29. The method of claim 28, wherein the level of p21 and PUMA is measured.

30. The method of claim 23, wherein compounds exhibiting a greater increase in post-exposure levels of the growth-suppressing marker in whole blood removed after administration than in whole blood removed before administration are identified as having cancer prophylaxis effects.

31. The method of claim 24, wherein compounds exhibiting no or a small increase in levels of the growth-suppressing marker in post-administration unexposed whole blood relative to pre-administration unexposed whole blood, and exhibiting a greater increase in post-exposure levels of the growth-suppressing marker in whole blood removed after administration than in whole blood removed before administration, are identified as having cancer prophylaxis effects with less risk of side effects.

## Patentansprüche

1. Verfahren zur Ermittlung der Krebsanfälligkeit bei einem Individuum, umfassend:
Vollblut des Individuums unter In-vitro-Bedingungen einem mutagenen Reizmittel aussetzen (Exposition),
Messen des Spiegels der wachstumsunterdrückenden Markersubstanz in Zellen, die von dem exponierten Vollblut abgeleitet sind, sowie in Zellen, die von nicht-exponiertem Vollblut des Individuums abgeleitet sind, und
Ermitteln der Krebsanfälligkeit des Individuums auf Grundlage des Unterschieds zwischen den Spiegeln der Markersubstanz in dem exponierten und dem nicht-exponierten Vollblut, wobei die Krebsanfälligkeit als erhöht ermittelt wird, wenn der Spiegel der wachstumsunterdrückenden Markersubstanz nach der Exposition nicht oder nur geringfügig ansteigt, wobei die wachstumsunterdrückende Markersubstanz aus p21, PUMA und BAX ausgewählt ist.

2. Verfahren nach Anspruch 1, wobei es sich bei den gemessenen Spiegeln um mRNA-Spiegel handelt.

3. Verfahren nach Anspruch 1, wobei es sich bei dem Individuum um einen Menschen handelt.

4. Verfahren nach Anspruch 1, wobei es sich bei dem mutagenen Reizmittel um ionisierende Strahlung handelt.

5. Verfahren nach Anspruch 1, wobei die Krebsanfälligkeit als verringert ermittelt wird, wenn der Spiegel der wachstumsunterdrückenden Markersubstanz nach der Exposition stark ansteigt.

6. Verfahren nach Anspruch 1, wobei der Spiegel einer Mehrzahl von wachstumsunterdrückenden Markersubstanzen gemessen wird.

7. Verfahren nach Anspruch 6, wobei der Spiegel von p21 und PUMA gemessen wird.

8. Verfahren zur Ermittlung der Krebsanfälligkeit bei einem Individuum, umfassend:
Durchführen einer Messung des Baselinedurchschnitts der Spiegel einer wachstumsunterdrückenden Markersubstanz im Vollblut von einer Mehrzahl von Mitgliedern der Spezies, der das Individuum angehört, nachdem das Vollblut unter In-vitro-Bedingungen einem mutagenen Reizmittel ausgesetzt wurde, Vollblut des Individuums unter In-vitro-Bedingungen einem mutagenen Reizmittel aussetzen,
Messen des Spiegels der wachstumsunterdrückenden Markersubstanz in Zellen, die von dem Vollblut nach der Exposition abgeleitet sind, und
Ermitteln der Krebsanfälligkeit des Individuums, wobei ein Spiegel, der die Messung des Baselinedurchschnitts übersteigt, auf ein niedrigeres Krebsrisiko hinweist und ein Spiegel, der die Messung des Baselinedurchschnitts unterschreitet, auf ein höheres Krebsrisiko hinweist, wobei die wachstumsunterdrückende Markersubstanz aus p21, PUMA und BAX ausgewählt ist.

9. Verfahren nach Anspruch 8, wobei es sich bei den gemessenen Spiegeln um mRNA-Spiegel handelt.

10. Verfahren nach Anspruch 8, wobei es sich bei dem Individuum um einen Menschen handelt.

11. Verfahren nach Anspruch 8, wobei es sich bei dem mutagenen Reizmittel um ionisierende Strahlung handelt.

12. Verfahren nach Anspruch 8, wobei der Spiegel einer Mehrzahl von wachstumsunterdrückenden Markersubstanzen gemessen wird.

13. Verfahren nach Anspruch 12, wobei der Spiegel von p21 und PUMA gemessen wird.

14. In-vitro-Verfahren zum Screening einer Verbindung auf krebsvorbeugende Wirkungen bei einem Individuum, umfassend:
Inkubieren von Vollblutproben des Individuums mit der Verbindung unter In-vitro-Bedingungen,
die inkubierten Vollblutproben und nicht-inkubierte Vollblutproben des Individuums unter In-vitro-Bedingungen einem mutagenen Reizmittel aussetzen,
Messen der Spiegel der wachstumsunterdrückenden Markersubstanz in Zellen, die von den exponierten Vollblutproben abgeleitet sind, sowie in Zellen, die von nicht-inkubiertem, nicht-exponiertem Vollblut des Individuums abgeleitet sind, und
Identifizieren von Verbindungen mit krebsvorbeugenden Wirkungen auf Grundlage der Unterschiede zwischen den Spiegeln der wachstumsunterdrückenden Markersubstanz in dem inkubierten Vollblut und dem nicht-inkubierten Vollblut nach der Exposition, wobei die wachstumsunterdrückende Markersubstanz aus p21, PUMA und BAX ausgewählt ist.

15. Verfahren nach Anspruch 14, wobei darüber hinaus die Spiegel der wachstumsunterdrückenden Markersubstanz in inkubiertem Vollblut gemessen werden, das keinem mutagenen Reizmittel ausgesetzt ist.

16. Verfahren nach Anspruch 14, wobei es sich bei den gemessenen Spiegeln um mRNA-Spiegel handelt.

17. Verfahren nach Anspruch 14, wobei es sich bei dem Individuum um einen Menschen handelt.

18. Verfahren nach Anspruch 14, wobei es sich bei dem mutagenen Reizmittel um ionisierende Strahlung handelt.

19. Verfahren nach Anspruch 14, wobei der Spiegel einer Mehrzahl von wachstumsunterdrückenden Markersubstanzen gemessen wird.

20. Verfahren nach Anspruch 19, wobei der Spiegel von p21 und PUMA gemessen wird.

21. Verfahren nach Anspruch 14, wobei Verbindungen mit einem größeren Anstieg der Spiegel der wachstumsunterdrückenden Markersubstanz in inkubiertem Vollblut als in nicht-inkubiertem Vollblut nach der Exposition als Verbindungen mit krebsvorbeugenden Wirkungen identifiziert werden.

22. Verfahren nach Anspruch 15, wobei Verbindungen mit keinem oder einem geringen Anstieg der Spiegel der wachstumsunterdrückenden Markersubstanz in inkubiertem, nicht-exponiertem Vollblut relativ zu nicht-inkubiertem, nicht-exponiertem Vollblut und einem größeren Anstieg der Spiegel der wachstumsunterdrückenden Markersubstanz in inkubiertem Vollblut als in nicht inkubiertem Vollblut nach der Exposition als Verbindungen mit krebsvorbeugenden Wirkungen mit einem geringeren Risiko von Nebenwirkungen identifiziert werden.

23. In-vitro-Verfahren zum Ermitteln, ob eine Verbindung eine Wirksamkeit bei der Krebsvorbeugung bei einem Individuum besitzt, umfassend:
die dem Individuum vor und nach der Verabreichung der Verbindung entnommenen Vollblutproben unter In-vitro-Bedingungen einem mutagenen Reizmittel aussetzen,
Messen der Spiegel einer wachstumsunterdrückenden Markersubstanz in den Zellen, die vor der Verabreichung von exponiertem Vollblut abgeleitet sind, und in Zellen, die vor der Verabreichung von nicht-exponiertem Vollblut abgeleitet sind, und
Ermitteln der krebsvorbeugenden Wirkungen der Verbindung auf Grundlage des Unterschieds zwischen den Spiegeln der wachstumsunterdrückenden Markersubstanz nach der Exposition in den vor bzw. nach der Verabreichung entnommenen Vollblutproben, wobei die wachstumsunterdrückende Markersubstanz aus p21, PUMA und BAX ausgewählt ist.

24. Verfahren nach Anspruch 23, wobei darüber hinaus die Spiegel der wachstumsunterdrückenden Markersubstanz in Vollblut gemessen werden, das nach der Verabreichung entnommen wurde und nicht dem mutagenen Reizmittel ausgesetzt wurde.

25. Verfahren nach Anspruch 23, wobei es sich bei den gemessenen Spiegeln um mRNA-Spiegel handelt.

26. Verfahren nach Anspruch 23, wobei es sich bei dem Individuum um einen Menschen handelt.

27. Verfahren nach Anspruch 23, wobei es sich bei dem mutagenen Reizmittel um ionisierende Strahlung handelt.

28. Verfahren nach Anspruch 23, wobei der Spiegel einer Mehrzahl von wachstumsunterdrückenden Markersubstanzen gemessen wird.

29. Verfahren nach Anspruch 28, wobei der Spiegel von p21 und PUMA gemessen wird.

30. Verfahren nach Anspruch 23, wobei Verbindungen mit einem größeren Anstieg, nach der Exposition, der Spiegel der wachstumsunterdrückenden Markersubstanz in Vollblut, das nach der Verabreichung entnommen wird, als in Vollblut, das vor der Verabreichung entnommen wird, als Verbindungen mit krebsvorbeugenden Wirkungen identifiziert werden.

31. Verfahren nach Anspruch 24, wobei Verbindungen mit keinem oder einem geringen Anstieg der Spiegel der wachstumsunterdrückenden Markersubstanz in nicht-exponiertem Vollblut nach der Verabreichung relativ zu nicht-exponiertem Vollblut vor der Verabreichung sowie einem größeren Anstieg der Spiegel, nach der Exposition, der wachstumsunterdrückenden Markersubstanz in Vollblut, das nach der Verabreichung entnommen wurde, als in Vollblut, das vor der Verabreichung entnommen wurde, als Verbindungen mit krebsvorbeugenden Wirkungen mit einem geringeren Risiko von Nebenwirkungen identifiziert werden.

## Revendications

1. Procédé permettant de déterminer la prédisposition au cancer chez un sujet comprenant les étapes consistant à :
exposer le sang total du sujet à un stimulant mutagène in vitro,
mesurer le niveau d'un marqueur d'inhibition de la croissance dans des cellules dérivées du sang total exposé et dans des cellules dérivées du sang total non exposé du sujet, et
déterminer la prédisposition du sujet au cancer à partir de la différence entre les niveaux de marqueur dans le sang total exposé et non exposé, la prédisposition au cancer étant considérée comme étant augmentée lorsque le niveau du marqueur d'inhibition de la croissance n'augmente pas ou augmente faiblement après l'exposition, le marqueur d'inhibition de la croissance étant choisi parmi les marqueurs p21, PUMA et BAX.

2. Procédé conforme à la revendication 1, selon lequel les niveaux mesurés sont des niveaux d'ARNm.

3. Procédé conforme à la revendication 1, selon lequel le sujet est un sujet humain.

4. Procédé conforme à la revendication 1, selon lequel le stimulant mutagène est une radiation ionisante.

5. Procédé conforme à la revendication 1, selon lequel la prédisposition au cancer est considérée comme étant diminuée lorsque le niveau de marqueur d'inhibition de la croissance augmente fortement après l'exposition.

6. Procédé conforme à la revendication 1, selon lequel le niveau de plusieurs marqueurs d'inhibition de la croissance est mesuré.

7. Procédé conforme à la revendication 1, selon lequel le niveau de marqueurs p21 et PUMA est mesuré.

8. Procédé de détermination de la prédisposition au cancer chez un sujet comprenant les étapes consistant à :
obtenir une mesure moyenne de référence de niveaux d'un marqueur d'inhibition de la croissance dans le sang total à partir d'un ensemble de membre de l'espèce du sujet après que ce sang total ait été exposé à un stimulant mutagène in vitro,
exposer le sang total du sujet à un stimulant mutagène in vitro,
mesure le niveau de marqueur d'inhibition de la croissance dans des cellules dérivées du sang total après exposition, et
déterminer la prédisposition du sujet au cancer, un niveau plus élevé que la mesure moyenne de référence indiquant un risque faible de cancer et un niveau supérieur à la mesure moyenne de référence indiquant un risque plus élevé de cancer, le marqueur d'inhibition de la croissance étant choisi parmi les marqueurs p21, PUMA et BAX.

9. Procédé conforme à la revendication 8, selon lequel les niveaux mesurés sont des niveaux d'ARNm.

10. Procédé conforme à la revendication 8, selon lequel le sujet est un sujet humain.

11. Procédé conforme à la revendication 8, selon lequel le stimulant mutagène est une radiation ionisante.

12. Procédé conforme à la revendication 8, selon lequel le niveau de plusieurs marqueurs d'inhibition de la croissance est mesuré.

13. Procédé conforme à la revendication 12, selon lequel le niveau des marqueurs p21 et PUMA est mesuré.

14. Procédé de criblage in vitro d'un composé pour en déterminer les effets prophylactiques contre le cancer chez un sujet comprenant les étapes consistant à :
mettre en incubation des échantillons de sang total du sujet avec le composé in vitro,
exposer les échantillons de sang total incubés et des échantillons de sang total non incubés du sujet à un stimulant mutagène in vitro,
mesurer des niveaux d'un marqueur d'inhibition de la croissance dans des cellules dérivées des échantillons de sang total ayant été exposé et dans des cellules dérivées de sang total non incubé n'ayant pas été exposé au sujet, et
identifier des composés ayant des effets prophylactiques contre le cancer à partir des différences entre les niveaux du marqueur d'inhibition de croissance dans le sang total incubé et dans le sang total non incubé après exposition le marqueur d'inhibition de la croissance étant choisi parmi les marqueurs p21, PUMA et BAX.

15. Procédé conforme à la revendication 14, selon lequel les niveaux du marqueur d'inhibition de la croissance sont en outre mesurés dans le sang total incubé et non exposé à un stimulant mutagène.

16. Procédé conforme à la revendication 14, selon lequel les niveaux mesurés sont des niveaux d'ARNm.

17. Procédé conforme à la revendication 14, selon lequel le sujet est un sujet humain.

18. Procédé conforme à la revendication 14, selon lequel le stimulant mutagène est une radiation ionisante.

19. Procédé conforme à la revendication 14, selon lequel le niveau de plusieurs marqueurs d'inhibition de la croissance est mesuré.

20. Procédé conforme à la revendication 19, selon lequel le niveau des marqueurs p21 et PUMA est mesuré.

21. Procédé conforme à la revendication 14, selon lequel les composés présentant une plus grande augmentation des niveaux de marqueur d'inhibition de la croissance après exposition dans le sang total ayant été incubé que dans le sang total n'ayant pas été incubé sont identifiés comme ayant des effets prophylactique contre le cancer.

22. Procédé conforme à la revendication 15, selon lequel des composés ne présentant pas ou ne présentant qu'une faible augmentation des niveaux de marqueur d'inhibition de la croissance dans le sang total incubé non exposé par rapport au sang total non incubé et non exposé et présentant une plus grande augmentation des niveaux du marqueur d'inhibition de la croissance après exposition dans le sang total incubé que dans le sang total non incubé sont identifiés comme ayant des effets de prophylaxie contre le cancer avec un risque inférieur d'effets secondaires.

23. Procédé pour déterminer in vitro si un composé est efficace dans la prophylaxie du cancer chez un sujet comprenant les étapes consistant à :
exposer des échantillons de sang total ayant été prélevés chez le sujet avant et après l'administration du composé, à un stimulant mutagène in vitro,
mesurer les niveaux d'un marqueur d'inhibition de la croissance dans les cellules dérivées d'échantillons de sang total exposé et dans des cellules dérivées de sang total non exposé prélevé avant l'administration, et
déterminer les effets du composé sur la prophylaxie du cancer à partir de la différence entre les niveaux de marqueur d'inhibition de la croissance après exposition dans les échantillons de sang total prélevés avant et après l'administration, le marqueur d'inhibition de la croissance étant choisi parmi les marqueurs p21, PUMA et BAX.

24. Procédé conforme à la revendication 23, selon lequel les niveaux de marqueur d'inhibition de la croissance sont en outre mesurés dans le sang total prélevé après administration n'ayant pas été exposé à un stimulant mutagène.

25. Procédé conforme à la revendication 23, selon lequel les niveaux mesurés sont des niveaux d'ARNm.

26. Procédé conforme à la revendication 23, selon lequel le sujet est un sujet humain.

27. Procédé conforme à la revendication 23, selon lequel le stimulant mutagène est une radiation ionisante.

28. Procédé conforme à la revendication 23, selon lequel le niveau de plusieurs marqueurs d'inhibition de la croissance est mesuré.

29. Procédé conforme à la revendication 28, selon lequel le niveau de marqueur p21 et PUMA est mesuré.

30. Procédé conforme à la revendication 23, selon lequel des composés présentant une plus grande augmentation des niveaux du marqueur d'inhibition de la croissance après exposition dans le sang total prélevé après l'administration que dans le sang total prélevé avant l'administration sont identifiés comme ayant des effets prophylactiques contre le cancer.

31. Procédé conforme à la revendication 24, selon lequel des composés ne présentant pas ou ne présentant qu'une faible augmentation des niveaux de marqueur d'inhibition de la croissance dans le sang total non exposé après administration que dans le sang total non exposé avant administration et présentant une plus grande augmentation des niveaux du marqueur d'inhibition de la croissance après exposition dans le sang total prélevé après administration que dans le sang total prélevé avant administration sont identifiés comme ayant des effets de prophylaxie contre le cancer avec un risque inférieur d'effets secondaires.


### SEQUENCE LISTING

<110> Hitachi Chemical Co., Ltd.
   Hitachi Chemical Research Center, Inc.
   Mitsuhashi, Masato
<120> METHOD OF MEASURING CANCER SUSCEPTIBILITY
<130> HITACHI.065VPC
<140> 60/574,248
   <141> 2004-05-25
<160> 9
<170> FastSEQ for Windows Version 4.0
<210> 1
   <211> 19
   <212> DNA
   <213> Artificial Sequence
<220>
   <223> Synthetic oligonucleotide primer sequence
<400> 1
   aaatgccaca cggctctca 19
<210> 2
   <211> 21
   <212> DNA
   <213> Artificial Sequence
<220>
   <223> Synthetic oligonucleotide primer sequence
<400> 2
   caagtgtctt cgtgtcgtgg g 21
<210> 3
   <211> 18
   <212> DNA
   <213> Artificial Sequence
<220>
   <223> Synthetic oligonucleotide primer sequence
<400> 3
   agccccctca ctcccaaa 18
<210> 4
   <211> 18
   <212> DNA
   <213> Artificial Sequence
<220>
   <223> Synthetic oligonucleotide primer sequence
<400> 4
   agccccctca ctcccaaa 18
<210> 5
   <211> 33
   <212> DNA
   <213> Artificial Sequence
<220>
   <223> Synthetic oligonucleotide probe sequence
<400> 5
   cagtggctag tggtgggtac tcaatgtgta ctt 33
<210> 6
   <211> 21
   <212> DNA
   <213> Artificial Sequence
<220>
   <223> Synthetic oligonucleotide probe sequence
<400> 6
   ccaaggccca gccctcacac a 21
<210> 7
   <211> 24
   <212> DNA
   <213> Artificial Sequence
<220>
   <223> Synthetic oligonucleotide primer sequence
<400> 7
   ccactggatt taagcagagt tcaa 24
<210> 8
   <211> 18
   <212> DNA
   <213> Artificial Sequence
<220>
   <223> Synthetic oligonucleotide primer sequence
<400> 8
   tccaacgagc ggcttcac 18
<210> 9
   <211> 26
   <212> DNA
   <213> Artificial Sequence
<220>
   <223> Synthetic oligonucleotide probe sequence
<400> 9
   cagcggccag tagcatctga ctttga 26
1727956
   052505
   ??
??
??
??
1